# EUROPEAN PATENT APPLICATION

(11) **EP 4 050 340 A1**
(43) Date of publication of application: **31.08.2022**
(21) Application number: 20878368.8
(22) Date of filing: 02.10.2020
(51) Int. Cl.: G01N 33/574, G01N 33/543

(54) **METHOD FOR SEPARATING AND DETECTING EXOSOMES, AND KIT FOR SEPARATION AND DETECTION THEREOF**

(30) Priority: 24.10.2019 JP 2019193707
(71) Applicant: JSR Corporation, Tokyo 105-8640 (JP); Keio University, Tokyo, 108-8345 (JP)
(72) Inventor: WAKUI, Seiki, Tokyo 105-8640 (JP); KAWAKAMI, Yutaka, Tokyo 160-8582 (JP); YAGUCHI, Tomonori, Tokyo 160-8582 (JP)
(74) Representative: TBK
(86) International application number: PCT/JP2020/037516
(87) International publication number: WO 2021/079717

(57) **Abstract**

What is provided is a method for separation and detection of exosomes, the method including: a complex formation step of bringing a biological sample into contact with a capture molecule, the capture molecule including a specific binding substance for an antigen expressed on a cancer cell surface, to form a complex of an exosome and the capture molecule; and a detection step of bringing the complex into contact with a detector molecule, the detector molecule including a specific binding substance for an antigen expressed on a cancer cell surface and a labeling substance, to detect the complex by using the detector molecule, in which the antigen expressed on a cancer cell surface for at least one of the capture molecule and the detector molecule is cell-surface vimentin.

## Description

### TECHNICAL FIELD

The present invention relates to a method for separation and detection of exosomes, and a kit for separation and detection of exosomes.

### BACKGROUND ART

It is known that cells release vesicles called exosomes, which are composed of a lipid bilayer membrane and include various cell contents such as proteins, nucleic acids, lipids, and sugars inside the vesicles, to the outside of the cells. Attention has been paid to exosomes as a material that serves as an important clue for understanding the pathological condition of a living organism, and research on various diseases including cancer is being conducted actively.

Meanwhile, vimentin is one of proteins important for cytoplasm formation and is known as a marker for intracellular epithelial-mesenchymal transition (EMT). Vimentin is overexpressed in cancer cells, and it has been found that there is a correlation between the expression level of vimentin and the progression of cancer. In a certain environment, vimentin migrates to the cell surface. Vimentin that has migrated to the cell surface is referred to as cell-surface vimentin (CSV).

It has been found that CSV is involved in autoimmune deficiency, viral infection, and cancer, and that since CSV is expressed in activated macrophages, platelets, and T lymphocytes that have undergone apoptosis, CSV is involved in inflammatory diseases. Recently, there has been reported a method for detecting peripheral circulating tumor cells (CTC) by combining an anti-CSV antibody with an antibody against the epithelial cell adhesion molecule (EpCAM, CD326), which is known as a diagnostic marker for carcinoma (see, for example, Non-Patent Document 1).

### PRIOR ART LITERATURE

### Non-Patent Documents

Non-Patent Document 1: Satelli A et al., "Circulating tumor cell enumeration using a combination of EpCAM and Cell-surface vimentin based methods for monitoring breast cancer therapeutic response.", Clin Chem., Vol. 61, Issue 1, pp. 259-266, 2015.

### DISCLOSURE OF INVENTION

### Problems to be Solved by the Invention

However, only a very small amount of CTC is present in blood, and it is difficult to detect an antigen marker for CTC intended for cancer diagnosis or companion diagnostics.

The present invention was achieved in view of the above-described circumstances and provides a method for separation and detection of exosomes, which can be utilized for cancer diagnosis or companion diagnostics, and a kit used for the method.

### Means for Solving the Problems

That is, the present invention includes the following aspects.
(1) A method for separation and detection of exosomes, the method including:
   a complex formation step of bringing a biological sample into contact with a capture molecule, the capture molecule including a specific binding substance for an antigen expressed on a cancer cell surface, to form a complex of an exosome and the capture molecule; and
   a detection step of bringing the complex into contact with a detector molecule, the detector molecule including a specific binding substance for an antigen expressed on a cancer cell surface and a labeling substance, to detect the complex by using the detector molecule,
   in which the antigen expressed on a cancer cell surface for at least one of the capture molecule and the detector molecule is cell-surface vimentin (CSV).
(2) The method according to (1), in which the specific binding substance for CSV is an anti-CSV antibody.
(3) The method according to (1) or (2), in which the specific binding substance for the antigen expressed on a cancer cell surface in the capture molecule is an anti-CSV antibody, and the specific binding substance for the antigen expressed on a cancer cell surface in the detector molecule is an anti-CSV antibody, an anti-EpCAM antibody, or an anti-PD-Ll antibody.
(4) The method according to (1) or (2), in which the specific binding substance for the antigen expressed on a cancer cell surface in the detector molecule is an anti-CSV antibody, and the specific binding substance for the antigen expressed on a cancer cell surface in the capture molecule is an anti-CSV antibody, an anti-EpCAM antibody, or an anti-PD-Ll antibody.
(5) The method according to any one of (1) to (4), in which the capture molecule is immobilized on a magnetic particle.
(6) The method according to any one of (1) to (5), the method further including, after the complex formation step and before the detection step,
   a washing step of adding a washing liquid to remove foreign substances other than the complex.
(7) The method according to any one of (1) to (6), in which in the detection step, the complex is brought into contact with the detector molecule, subsequently a washing liquid is added to remove foreign substances other than the complex to which the detector molecule is bound, and then the complex is detected by using the detector molecule.
(8) A kit for separation and detection of exosomes, the kit including:
   a capture molecule including a specific binding substance for an antigen expressed on a cancer cell surface; and
   a detector molecule including a specific binding substance for an antigen expressed on a cancer cell surface and a labeling substance,
   in which the antigen expressed on a cancer cell surface for at least one of the capture molecule and the detector molecule is CSV.
(9) The kit according to (8), in which the specific binding substance for the antigen expressed on cancer cell surface is an anti-CSV antibody.
(10) The kit according to (8) or (9), in which the specific binding substance for the antigen expressed on a cancer cell surface in the capture molecule is an anti-CSV antibody, and the specific binding substance for the antigen expressed on a cancer cell surface in the detector molecule is an anti-CSV antibody, an anti-EpCAM antibody, or an anti-PD-Ll antibody.
(11) The kit according to (8) or (9), in which the specific binding substance for the antigen expressed on a cancer cell surface in the detector molecule is an anti-CSV antibody, and the specific binding substance for the antigen expressed on a cancer cell surface in the capture molecule is an anti-CSV antibody, an anti-EpCAM antibody, or an anti-PD-Ll antibody.
(12) The kit according to any one of (8) to (11), in which the capture molecule is immobilized on a magnetic particle.
(13) The kit according to any one of (8) to (12), in which the kit is used for diagnosing cancer.
(14) The kit according to any one of (8) to (13), in which the kit is used for diagnosing early-stage cancer.
(15) The kit according to any one of (8) to (14), in which the kit is used for diagnosing lung cancer.
(16) The kit according to any one of (8) to (12), in which the kit is used for companion diagnostics.

### Effects of the Invention

According to the method for separation and detection of exosomes and the kit for separation and detection of exosomes according to the above-described embodiment, exosomes can be separated and detected, and the method and the kit can be used for cancer diagnosis or companion diagnostics.

### BRIEF DESCRIPTION OF THE DRAWINGS

Fig. 1 is a graph showing the results for measuring CSV-positive exosomes in lung cancer patient specimens or healthy subject specimens in Analysis Example 1. In the diagram, the numerical value in the parentheses indicates the number of cases.
Fig. 2 is a graph showing an ROC curve created from the measurement results shown in Fig. 1.
Fig. 3 is a graph showing the results for measuring CSV- and EpCAM-positive exosomes in lung cancer patient specimens or healthy subject specimens in Analysis Example 2. In the diagram, the numerical value in the parentheses indicates the number of cases.
Fig. 4 is a graph showing an ROC curve created from the measurement results shown in Fig. 3.
Fig. 5 is a graph showing the results for measuring CSV- and PD-L1-positive exosomes in lung cancer patient specimens or healthy subject specimens in Analysis Example 3. In the diagram, the numerical value in parentheses indicates the number of cases.
Fig. 6 is a graph showing an ROC curve created from the measurement results shown in Fig. 5.
Fig. 7A is a graph obtained by classifying the measurement results shown in Fig. 1 according to the type of lung cancer. In the diagram, the numerical value in the parentheses indicates the number of cases.
Fig. 7B is a graph obtained by classifying the measurement results shown in Fig. 3 according to the type of lung cancer. In the diagram, the numerical value in the parentheses indicates the number of cases. However, (12000) of Group Ad represents a Signal/Noise value.
Fig. 7C is a graph obtained by classifying the measurement results shown in Fig. 5 according to the type of lung cancer. In the diagram, the numerical value in the parentheses indicates the number of cases.
Fig. 8A is a graph obtained by classifying the measurement results shown in Fig. 1 according to the pathological stage of lung cancer. In the diagram, the numerical value in the parentheses indicates the number of cases.
Fig. 8B is a graph obtained by classifying the measurement results shown in Fig. 3 according to the pathological stage of lung cancer. In the diagram, the numerical value in the parentheses indicates the number of cases. However, (12000) of Group III represents a Signal/Noise value.
Fig. 8C is a graph obtained by classifying the measurement results shown in Fig. 5 according to the pathological stage of lung cancer. In the diagram, the numerical value in the parentheses indicates the number of cases.
Fig. 9A is a graph obtained by classifying the measurement results shown in Fig. 1 according to the pathological stage of adenocarcinoma or squamous cell carcinoma. In the diagram, the numerical value in the parentheses indicates the number of cases.
Fig. 9B is a graph obtained by classifying the measurement results shown in Fig. 3 according to the pathological stage of adenocarcinoma or squamous cell carcinoma. In the diagram, the numerical value in the parentheses indicates the number of cases. However, (12000) of Group Adenocarcinoma III represents a Signal/Noise value.
Fig. 9C is a graph obtained by classifying the measurement results shown in Fig. 5 according to the pathological stage of adenocarcinoma or squamous cell carcinoma. In the diagram, the numerical value in the parentheses indicates the number of cases.
Fig. 10 is a graph showing the measurement results of CSV-positive exosomes, CSV- and EpCAM-positive exosomes, and CSV- and GPC3-positive exosomes in lung cancer patient specimens or healthy subject specimens in Example 4.
Fig. 11 is a graph showing the measurement results of CSV- and PD-L1-positive exosomes in the CSV/PD-L1 evaluation system and the PD-L1/CSV evaluation system in Example 5.

### EMBODIMENTS FOR CARRYING OUT THE INVENTION

Hereinafter, the present invention will be described in more detail by way of embodiments; however, the present invention is not intended to be limited to the following embodiments.

### <Method for separation and detection of exosomes>

According to an embodiment, the present invention provides a method for separation and detection of exosomes, the method including a complex formation step and a detection step.

In the complex formation step, a biological sample is brought into contact with a capture molecule that includes a specific binding substance for an antigen expressed on a cancer cell surface, to form a complex of an exosome and the capture molecule.

In the detection step, the complex is brought into contact with a detector molecule that includes a specific binding substance for an antigen expressed on a cancer cell surface and a labeling substance, to detect the complex by using the detector molecule.

The antigen expressed on the cancer cell surface for at least one of the capture molecule and the detector molecule is cell-surface vimentin (CSV).

The inventors found that CSV is mainly expressed on the surface of exosomes released from cancer cells, thus completing the present invention.

In conventional methods for detecting an antigen marker for CTC, since 10 mL of blood includes only about 1 or more and 10 or fewer CTCs, a large amount of blood sample is required for detection, and it is difficult to detect the antigen marker for CTC for the purpose of cancer diagnosis. Particularly, although promptness and accuracy of diagnosis are required in cancer diagnosis and companion diagnostics, detection of an antigen marker for CTC cannot satisfy the needs.

On the other hand, in the method for separation and detection of exosomes according to the embodiment of the present invention (hereinafter, may be simply described as "method for separation and detection of the present embodiment"), since exosomes in an amount sufficient for measurement are included in a sample of about several dozens of µL of blood, exosomes are suitable as a detection target for cancer diagnosis or companion diagnostics. Furthermore, among the antigen markers present in one exosome, exosomes released from cancer cells can be specifically separated by targeting CSV and a known cancer antigen marker in combination. As a result, the accuracy of cancer diagnosis can be increased. In addition, companion diagnostics can be performed by selecting a cancer antigen that is a target of a therapeutic agent as a known cancer antigen marker to be combined with CSV.

Next, each step of the method for separation and detection of the present embodiment will be described in detail.

### [Complex formation step]

In the complex formation step, a biological sample is brought into contact with a capture molecule including a specific binding substance for an antigen expressed on a cancer cell surface. Through this contact, a complex of an exosome and the capture molecule is formed.

Examples of the biological sample include various liquids such as a body fluid, a cell culture supernatant, and a tissue cell disruption fluid. Above all, a body fluid or a cell culture supernatant is preferred. Examples of the body fluid include whole blood, serum, blood plasma, various blood cells, blood clots, blood platelets, as well as urine, semen, breast milk, sweat, interstitial fluid, interstitial lymph fluid, bone marrow fluid, tissue fluid, saliva, gastric juice, synovial fluid, pleural effusion, bile, ascites fluid, and amniotic fluid. Above all, blood plasma is preferred. Incidentally, blood plasma may be treated with an anticoagulant such as citric acid, heparin, or EDTA.

The species from which the biological sample is derived is not particularly limited, and examples thereof include human, mouse, rat, rabbit, dog, pig, sheep, cattle, horse, camel, and monkey. Among these, a human-derived sample is preferred. Furthermore, regarding the combination of the species and a body fluid, human-derived blood plasma is preferred.

Regarding the biological sample, a sample that has been pretreated in advance by adding the sample to a known buffer or the like may be used; however, a sample taken in from a living organism can be used as it is. That is, according to the method for separation and detection of the present embodiment, selective separation of exosomes can be conveniently carried out without performing a pretreatment by a PEG precipitation method, an isolation method using an ultracentrifuge, or the like.

The amount of the biological sample can be appropriately adjusted; however, the amount may be, for example, 1 µL or more and 50 µL or less and may be, for example, 1 µL or more and 20 µL or less.

The capture molecule used in the complex formation step includes a specific binding substance for an antigen expressed on a cancer cell surface. Since the exosomes released from cancer cells have an antigen expressed on a cancer cell surface, the exosomes released from cancer cells can be captured by using a specific binding substance for the antigen.

The antigen expressed on a cancer cell surface means an antigen that is known to be more highly expressed in cancer cells than in normal cells, or an antigen that is known to be specifically expressed only in cancer cells. Examples of such an antigen include, but are not limited to, cell-surface vimentin (CSV), epithelial cell adhesion molecule (EpCAM, CD326), programmed cell death ligand-1 (PD-L1), glypican-3 (GPC3), glypican-1 (GPC1), carcinoembryonic antigen (CEA), CD4, CD5, CD16, CD19, CD20, CD22, CD30, CD33, CD38, CD44, CD52, CD123, CD171, Cancer Antigen 125, Claudin18, receptor-type tyrosine kinase ROR1, sialyl. Le^{x}-i (SLX), Sialyl Lewis A (CA19-9), immune co-stimulatory protein B7-homolog6 (B7H6), cell surface glycoprotein (CS-1), receptor-type tyrosine kinase 3 (FLT3), prostate-specific membrane antigen (PSMA), B-cell maturation antigen (BCMA), Wilms tumor 1 (WT1), NY-ESO-1, Caveolin-1, Fas ligand (FasL), TNF-related apoptosis-inducing ligand (TRAIL), Galectine3, CD151, Tetraspanin 8, epithelial growth factor receptor (EGFR), HER2, Ribophorin 2 (RPN2), CD44, and TGF-β.

Information on the base sequences and amino acid sequences of the above-described various antigens expressed on a cancer cell surface in human can be obtained from a database such as Genbank. The amino acid sequence of human CSV (as vimentin) is disclosed as, for example, Genbank Accession No. NP_003371.2. The amino acid sequence of human EpCAM is disclosed as, for example, Genbank Accession No. NP_002345.2. The amino acid sequence of human PD-L1 is disclosed as, for example, Genbank Accession No. NP_054862.1. The amino acid sequence of human GPC3 is disclosed as, for example, Genbank Accession No. NP_001158089.1.

The capture molecule includes a specific binding substance for the above-described antigen.

Examples of the specific binding substance include a substance that specifically binds to an antigen. Examples of the substance that specifically binds to an antigen include an antibody and an aptamer.

The antibody may be an antibody having an ability to bind to an antigen sequence (epitope) on cancer cells, and specific examples thereof include an anti-CSV antibody, an anti-EpCAM antibody, an anti-PD-Ll antibody, and an anti-GPC3 antibody. These antibodies may be monoclonal antibodies or may be polyclonal antibodies; however, it is preferable that the antibodies are monoclonal antibodies. In addition, these antibodies may also be bispecific antibodies. Examples of the class of the antibodies include IgG and IgM; however, IgG is more preferred. Furthermore, the antibodies may be chimeric antibodies such as humanized antibodies. The antibodies may be antibody derived from, for example, animal species, including mammals such as mouse, rabbit, cattle, pig, horse, sheep, and goat; birds such as chickens; and human, and there are no particular limitations. The antibody may be prepared from, for example, blood serum derived from the above-described animal species by a conventionally known method, the antibody may be prepared from a vector synthesized using known antibody gene sequence information, or a commercially available antibody may be used. Examples of a commercially available anti-CSV monoclonal antibody include a mouse anti-human CSV monoclonal antibody (isotype: IgG2b, κ) manufactured by Abnova Corporation.

Furthermore, an antibody fragment can also be used as the antibody. Examples of the antibody fragment include Fv, Fab, Fab', F(ab')2, rIgG, and scFv.

An aptamer is a substance having an ability to specifically bind to a target substance. Examples of the aptamer include a nucleic acid aptamer and a peptide aptamer. A nucleic acid aptamer having an ability to specifically bind to an antigen can be screened by, for example, a method of systematic evolution of ligand by exponential enrichment (SELEX). Furthermore, a peptide aptamer having an ability to specifically bind to an antigen can be screened by, for example, a Two-hybrid method using yeast.

Furthermore, when the antigen is CSV, Nkp46 protein, which is known to have an ability to bind to CSV, or the like can also be used as the specific binding substance.

It is preferable that the capture molecule is immobilized on a solid-phase carrier.

Examples of the material of the solid-phase carrier include polymer compounds such as polystyrenes, polyethylenes, polypropylenes, polyesters, poly(meth)acrylonitriles, a styrene-butadiene copolymer, poly(meth)acrylic acid esters, fluororesins, crosslinked dextran, and a polysaccharide; glass; metals; magnetic materials; a resin composition including a magnetic material; and combinations of these.

The shape of the solid-phase carrier is not particularly limited, and examples thereof include a tray shape, a spherical shape, a particulate shape, a fibrous shape, a rod shape, a disc shape, a container shape, and a microchannel.

In the present step, from the viewpoint of the ease of solid-liquid separation and washing, it is preferable to use magnetic particles as the solid-phase carrier.

Examples of the magnetic particles include magnetic fine particles formed from triiron tetraoxide (Fe₃O₄), iron sesquioxide (γ-Fe₂O₃), various ferrites, and metals such as iron, manganese, nickel, cobalt, and chromium; magnetic fine particles formed from alloys of these metals; and magnetic particles including these magnetic fine particles in a resin. Examples of the resin include a hydrophobic polymer and a hydrophilic polymer.

Above all, magnetic particles including a magnetic substance in a resin are preferred, and magnetic particles in which a polymer layer is formed on the surface of mother particles including superparamagnetic fine particles are more preferred. For example, magnetic particles in which a hydrophobic first polymer layer is formed on the surface of a mother particle including superparamagnetic fine particles, a second polymer layer having at least a glycidyl group on the surface is formed on the first polymer layer, and a polar group is introduced by chemically modifying the glycidyl group, as described in Japanese Unexamined Patent Application, First Publication No. 2008-32411 (Reference Document 1), may be mentioned.

Here, regarding the superparamagnetic fine particles, a representative example is iron oxide-based fine particles having a particle diameter of 20 nm or less (preferably, a particle diameter of 5 nm or more and 20 nm or less), while examples include ferrite represented by the formula: XFe₂O₄ (X = Mn, Co, Ni, Mg, Cu, Li_{0.5}Fe_{0.5}, or the like), magnetite represented by the formula: Fe₃O₄, and γ-Fe₂O₃, and from the viewpoint that the saturation magnetization is strong and the residual magnetization is small, it is preferable to include either γ-Fe₂O₃ or Fe₃O₄.

Furthermore, monomers for forming the hydrophobic first polymer layer are roughly classified into a monofunctional monomer and a crosslinkable monomer.

Examples of the monofunctional monomer include aromatic vinyl-based monomers such as styrene, α-methylstyrene, and halogenated styrene; and ethylenically unsaturated carboxylic acid alkyl ester-based monomers such as methyl acrylate, methyl methacrylate, ethyl acrylate, ethyl methacrylate, stearyl acrylate, stearyl methacrylate, cyclohexyl acrylate, cyclohexyl methacrylate, isobornyl acrylate, and isobornyl methacrylate.

Examples of the crosslinkable monomer include polyfunctional (meth)acrylates such as ethylene glycol diacrylate, ethylene glycol dimethacrylate, trimethylolpropane triacrylate, trimethylolpropane trimethacrylate, pentaerythritol triacrylate, pentaerythritol trimethacrylate, dipentaerythritol hexaacrylate, and dipentaerythritol hexamethacrylate; and conjugated diolefins such as butadiene and isoprene; as well as divinylbenzene, diallyl phthalate, allyl acrylate, and allyl methacrylate.

Furthermore, the monomer for forming the second polymer layer has a main purpose of introducing a functional group onto the particle surface and includes a glycidyl group-containing monomer. The content of the glycidyl group-containing monomer is preferably 20% by mass or more among the monomers for forming the second polymer layer. Here, examples of the copolymerizable monomer including a glycidyl group include glycidyl acrylate, glycidyl methacrylate, and allyl glycidyl ether.

It is preferable that the polar group that is introduced by chemically modifying the glycidyl group of the second polymer layer is a functional group capable of reacting with a specific binding substance for an antigen expressed on a cancer cell surface, and it is more preferable that the polar group includes one or more atoms of at least one kind selected from the group consisting of an oxygen atom, a nitrogen atom, and a sulfur atom. Above all, an amino group, an aldehyde group, a carboxy group, or an active ester group is more preferred. Particularly, when the second polymer layer of the magnetic particles has the above-described polar group and a 2,3-dihydroxypropyl group, the binding property of the second polymer layer with the specific binding substance for an antigen expressed on a cancer cell surface (particularly, an antibody to an antigen expressed on a cancer cell surface) is satisfactory.

As a method for causing the specific binding substance for an antigen expressed on a cancer cell surface to bind to a solid-phase carrier, a physical adsorption method; a chemical bonding method such as a covalent bonding method or an ionic bonding method; and the like are used. Examples of the physical adsorption method include a method of directly immobilizing the specific binding substance for an antigen expressed on a cancer cell surface on a solid-phase carrier; and a method of chemically bonding the specific binding substance to another protein such as albumin and then adsorbing the specific binding substance to be immobilized. Examples of the chemical bonding method include a method of directly binding the specific binding substance for an antigen expressed on a cancer cell surface, onto a solid-phase carrier by utilizing a functional group that has been introduced onto the surface of the solid-phase carrier and is capable of reacting with the specific binding substance; a method of introducing a spacer molecule (a carbodiimide compound or the like) by chemical bonding between a solid-phase carrier and a specific binding substance for an antigen expressed on a cancer cell surface, and then binding the specific binding substance to the spacer molecule; and a method of causing the specific binding substance for an antigen expressed on a cancer cell surface, to bind to another protein such as albumin, and then chemically bonding the protein to a solid-phase carrier.

The complex formation step may also be carried out, if necessary, using a salt, a protein such as albumin, a surfactant, or the like, in addition to each of the above-described components.

The reaction temperature in the complex formation step is usually in the range of 2°C or higher and 42°C or lower, and the reaction time is usually 5 minutes or longer and 24 hours or shorter. When the reaction is carried out at 2°C or higher and 8°C or lower, the reaction time is preferably 8 hours or longer and 30 hours or shorter, and when the reaction is carried out at room temperature (20°C) or higher and 42°C or lower, the reaction time is preferably 5 minutes or longer and 4 hours or shorter.

In the complex formation step, the pH in the system is not particularly limited; however, the pH is preferably in the range of pH 5 or higher and 10 or lower, and more preferably in the range of pH 6 or higher and 8 or lower. In order to maintain the intended pH, a buffer solution is usually used, and examples thereof include a phosphate buffer solution, a tris(hydroxymethyl)aminomethane buffer solution, a HEPES buffer solution, and a MES buffer solution.

### [Detection step]

In the detection step, the above-described complex is brought into contact with a detector molecule including a specific binding substance for an antigen expressed on a cancer cell surface and a labeling substance. Through this contact, the detector molecule is bound to the exosome in the complex, and the complex is detected by using the detector molecule.

The detector molecule includes a specific binding substance for an antigen expressed on a cancer cell surface and a labeling substance. Above all, it is preferable that the detector molecule includes a specific binding substance for an antigen expressed on a cancer cell surface, the specific binding substance having a labeling substance bound thereto.

Examples of the specific binding substance for an antigen expressed on a cancer cell surface include substances similar to those mentioned as examples in the section "Capture molecule". The specific binding substance for the capture molecule and the specific binding substance for the detector molecule may be the same or may be different. When the specific binding substance of the capture molecule and the specific binding substance of the detector molecule are the same, the specific binding substance may be a specific binding substance for CSV, and the specific binding substance is preferably an anti-CSV antibody.

Furthermore, when the specific binding substance of the capture molecule or the specific binding substance of the detector molecule is an antibody, substances composed of bispecific antibodies may be used for those, or a mixture of a plurality of antibodies for different antigens may be used.

Furthermore, it is preferable that the specific binding substance of the capture molecule is an anti-CSV antibody and the specific binding substance of the detector molecule is an anti-CSV antibody, an anti-EpCAM antibody, or an anti-PD-Ll antibody.

Furthermore, it is also preferable that the specific binding substance of the detector molecule is an anti-CSV antibody and the specific binding substance of the capture molecule is an anti-CSV antibody, an anti-EpCAM antibody, or an anti-PD-Ll antibody.

When the specific binding substance of the capture molecule and the specific binding substance of the detector molecule constitute the above-described combinations, exosomes can be detected with high accuracy as disclosed in the Examples that will be described later.

Examples of the labeling substance include charged molecules such as biotin, avidin, streptavidin, NeutrAvidin, glutathione-S-transferase, glutathione, alkaline phosphatase (ALP), a fluorescent dye, polyethylene glycol, and mellitic acid. When the labeling substance is ALP, exosomes can be detected with high accuracy by adding an ALP luminescent substrate. Examples of the ALP luminescent substrate include p-nitrophenyl phosphate (PNP), 4-methylumbelliferyl phosphate (4MUP), 3-(2'-spiroadamantane) 4-methoxy-4-(3 "-phosphoryloxy)phenyl-1,2-dioxetane (AMPPD), Disodium 3-(4-methoxyspiro{1,2-dioxetane-3,2'-(5'-chloro)tricyclo[3.3.1.1^{3,7}]decan}-4-yl)phenyl phosphate (CSPD), 2-chloro-5-(4-methoxyspiro{1,2-dioxetane-3,2'-(5'-chloro)-tricyclo[3.3.1.1^{3,7}]decan}-4-yl)-1-phenyl phosphate disodium (CDP-Star).

The detection method for the detection step may be carried out by an immunoassay method, examples thereof include an enzyme immunoassay (EIA), ELISA, chemiluminescence enzyme immunoassay (CLEIA), fluorescence immunoassay (FIA), radioimmunoassay (RIA), luminescence immunoassay, immunoblotting, and Western blotting, and when the specific binding substance is an antibody, the CLEIA method or the ELISA method is preferred from the viewpoint that the antibody can be conveniently detected with high sensitivity. Examples of the ELISA method include a competition method and a sandwich method.

An example of the method for separation and detection in the case of using the CLEIA method will be shown. First, an antibody against an antigen expressed on a cancer cell surface is bound to a solid-phase carrier to immobilize a capture molecule, and then a biological sample is brought into contact to form a complex. To this, a detector molecule including an antibody against an antigen expressed on a cancer cell surface, the detector molecule having a labeling substance bound thereto, is added to form a further complex. Then, by detecting the quantity of labeling in the formed complex, a signal derived from the exosomes included in the biological sample can be measured.

The method for separation and detection of the present embodiment can include other steps in addition to the complex formation step and the detection step.

For example, a washing step may be provided after the complex formation step and before the detection step.

In the washing step, the complex of the exosome and the capture molecule formed in the complex formation step is washed. As a result, foreign substances such as unreacted components are removed.

The washing step usually employs different methods depending on the shape of the solid-phase carrier, which is a constituent component of the capture molecule. When the solid-phase carrier is in the form of particles such as magnetic particles, for example, a method of performing washing by dispersing magnetic particles in a washing liquid, or a method of washing by dipping while having magnetic particles collected may be mentioned. On the other hand, when the solid-phase carrier is in the form of a microplate, a method of performing washing by repeatedly dispensing and suctioning a washing liquid onto the plate may be mentioned.

When the solid-phase carrier is magnetic particles, it is preferable that the washing step includes a magnetic collecting step of collecting the magnetic particles by means of magnetic force to separate the magnetic particles from the liquid phase, and a dispersing step of dispersing the magnetic particles separated in the magnetic collecting step into the washing liquid. As a result, unreacted substances and foreign substances in the sample can be more efficiently washed, separated, and removed from the surface of the magnetic particles.

Specifically, washing may be carried out by repeatedly performing an operation of applying a magnetic field to the reaction vessel, attaching magnetic particles to the wall of the reaction vessel to collect the magnetic particles, subsequently removing the reaction supernatant, further adding a washing liquid thereto as necessary, similarly applying a magnetic field in the same manner, and then removing the supernatant.

Regarding the washing liquid, a washing liquid including a surfactant and the buffer solution mentioned in the above-mentioned complex formation step is preferred. The pH of the washing liquid is preferably in the range of pH 5 or higher and 10 or lower, and more preferably in the range of pH 6 or higher and 8 or lower. Specific examples thereof include Tris Buffered Saline (TBS) including 0.1% by mass of Tween 20 with respect to the total mass of the solution.

Furthermore, in the detection step, the complex may be brought into contact with a detector molecule including a specific binding substance for an antigen expressed on a cancer cell surface and a labeling substance, and then a washing liquid may be added thereto to wash the complex having the detector molecule bound thereto. As a result, unreacted substances and foreign substances in the sample can be removed, and the complex can be detected with higher sensitivity by using the detector molecule.

Furthermore, in the method for separation and detection of the present embodiment, as shown in the Examples that will be described later, the detection sensitivity of exosomes can be further increased by using a combination of two or more kinds of capture molecules and detector molecules and carrying out each step in parallel.

### <Kit for separation and detection of exosomes>

A kit for separation and detection of exosomes according to an embodiment of the present invention (hereinafter, may be simply described as "kit for separation and detection of the present embodiment") is equipped with a capture molecule and a detector molecule. The capture molecule includes a specific binding substance for an antigen expressed on a cancer cell surface. The detector molecule includes a specific binding substance for an antigen expressed on a cancer cell surface and a labeling substance. The antigen expressed on a cancer cell surface in at least either the capture molecule or the detector molecule is CSV.

The capture molecule and the detector molecule are similar to those mentioned as examples in the section "Method for separation and detection of exosomes".

The kit for separation and detection of the present embodiment can further include a washing liquid in addition to the capture molecule and the detector molecule. The washing liquid is similar to that mentioned as examples in the section "Method for separation and detection of exosomes".

### <Use applications>

The method for separation and detection of the present embodiment and the kit for separation and detection are suitably used for the diagnosis of cancer, particularly the diagnosis of early-stage cancer. That is, according to an embodiment, the present invention provides a method for determining cancer, the method including a determination step of using a biological sample collected from an examinee to compare a signal derived from exosomes detected by the above-described method for separation and detection with an exosome-derived signal that serves as a background in a biological sample collected from a control subject, and when the signal derived from exosomes in the sample of the examinee is stronger than the signal derived from exosomes in the sample of the control subject (background), determining that the examinee has developed cancer.

Furthermore, the kit for separation and detection can be said differently as a kit for cancer diagnosis.

Examples of the cancer to be diagnosed include colorectal cancer, breast cancer, uterine body cancer, cervical cancer, ovarian cancer, pancreatic cancer, stomach cancer, esophageal cancer, liver cancer, lung cancer, kidney cancer, and skin cancer; however, above all, lung cancer is particularly preferred.

In the determination step, whether an examinee has developed cancer can be determined by performing a statistical analysis of a signal derived from exosomes in an examinee's sample as measured in the above-described detection step on the basis of a signal derived from exosomes in a control subject's sample, to perform a comparison. For example, when the signal derived from exosomes in the examinee's sample is stronger than the signal derived from exosomes in the control subject's sample, it is determined that there is a high possibility of having developed cancer. Incidentally, the control subject in the determination step is a person of the same age and same sex as the examinee, the person being an average of those who have not developed cancer, and the exosome-derived signal for the control subject may be measured together with the exosome-derived signal for the examinee, or a statistical value obtained from values that have been separately measured in advance may be used.

Furthermore, the method for separation and detection of the present embodiment and the kit for separation and detection are suitably used for companion diagnostics for selecting a cancer therapeutic agent. That is, according to an embodiment, the present invention provides a method for selecting a cancer therapeutic agent, the method including a selection step of using a biological sample collected from an examinee to compare a signal derived from exosomes that are positive for a particular cancer antigen detected by the above-described method for separation and detection with a signal derived from exosomes that are positive for a particular cancer antigen in a biological sample collected from a control subject, and when the signal derived from exosomes that are positive for the particular cancer antigen in the examinee is comparable to the signal derived from exosomes that are positive for the particular cancer antigen in the control subject, or is stronger than the signal derived from exosomes that are positive for the particular cancer antigen in the control subject, selecting a cancer therapeutic agent that targets the particular cancer antigen as a molecular target, as a cancer therapeutic agent to be used for the examinee.

Furthermore, the kit for separation and detection can be said differently as a kit for companion diagnostics of a cancer therapeutic agent.

In the selection step, a cancer therapeutic agent to be used for an examinee can be selected by performing a statistical analysis of a signal derived from exosomes in an examinee's sample as measured in the above-described detection step on the basis of a signal derived from exosomes in a control subject's sample, to perform a comparison. For example, when the signal derived from exosomes that are positive for a particular cancer antigen in the examinee's sample is comparable to or higher than the signal derived from exosomes that are positive for the cancer antigen in the control subject's sample, a cancer therapeutic agent that targets the cancer antigen as a molecular target can be selected as a cancer therapeutic agent to be used for the examinee. Incidentally, the control subject in the selection step is a person of the same age and same sex as the examinee, the person being an average of those cancer patients who have obtained a therapeutic effect with the candidate cancer therapeutic agent, and regarding the signal derived from exosomes that are positive for a particular cancer antigen in the control subject, a statistical value obtained from the values measured before medication of the control subject can be used.

Furthermore, since exosomes are secreted from various cells, for example, immune system cells and various cancer cells, drug efficacy in patients can be evaluated by measuring the changes in the exosomes in blood (in addition to the increase or decrease in abundance, also including the fluctuation in the amount of membrane proteins) before and after administration of a cancer therapeutic agent. That is, according to an embodiment, the present invention provides a method for evaluating the drug efficacy of a cancer therapeutic agent, the method including an evaluation step of using a biological sample collected from an examinee before administration and after administration of the cancer therapeutic agent, and with regard to signals derived from exosomes detected by the above-described method for separation and detection, evaluating that when a signal derived from exosomes in the biological sample collected from the examinee after administration of the cancer therapeutic agent is lower than a signal derived from exosomes in the biological sample collected from the examinee before administration of the cancer therapeutic agent, there is a high possibility of the cancer therapeutic agent exhibiting drug efficacy.

In the evaluation step, a statistical analysis of the signal derived from exosomes detected by the above-described method for separation and detection is performed on the basis of the signal derived from exosomes in the biological sample collected from the examinee before administration of the cancer therapeutic agent, and a comparison is performed. The analysis method is not particularly limited, and a known method can be used. Furthermore, in the subsequent determination, for example, when the signal derived from exosomes in the biological sample collected from the examinee after administration of the cancer therapeutic agent is lower compared with the signal derived from exosomes in the biological sample collected from the examinee before administration, it can be evaluated that there is a high possibility that the cancer therapeutic agent has an effect of suppressing cancer.

### EXAMPLES

Hereinafter, the present invention will be described in detail by way of Examples; however, the present invention is not intended to be limited to these Examples.

### <Preparation of sample>

### [Preparation of heparin plasma specimen]

Blood was collected from a healthy subject or a lung cancer patient into a blood-collecting tube containing heparin sodium and was centrifuged at room temperature at 1500×g to 1800×g for 15 minutes, and the supernatant was collected into a separate container and used as a heparin plasma specimen. The heparin plasma specimen was stored at -80°C until use.

### <Method for measuring exosomes>

The measurement of exosomes in Examples and Comparative Examples that will be described later was performed by measuring the emission intensity by chemiluminescence enzyme immunoassay (CLEIA method). The measurement method used in each Example will be described in detail below.

### [Measurement method A]

### (Measurement of emission intensity by CLEIA method in CSV/CSV evaluation system)

Each heparin plasma specimen was appropriately diluted in advance with a 50 mM Tris buffer containing 2% (w/v) BSA and 150 mM NaCl, and this was used as a sample solution. Onto a 96-well white microplate (manufactured by Corning, Inc.), 25 µL/well of a dispersion liquid of 0.05% (w/v) antibody-bound magnetic particles (Magnosphere MS300/Carboxyl (product No.: MSP-S300-CA, manufactured by JSR Life Sciences, LLC) was sensitized with the antibody by JSR Corporation) having an anti-CSV antibody (catalogue No.: H00007431-M08, manufactured by Abnova Corporation) bound thereto was added, and next, 25 µL/well of a sample solution was added thereto. In addition, 25 µL/well of a solution containing an alkaline phosphatase-labeled anti-CSV antibody (catalogue No.: H00007431-M08, product manufactured by Abnova Corporation was labeled by JSR Corporation) was added thereto.

Subsequently, the 96-well white microplate was stirred at 25°C for 20 minutes, and next, while magnetic particles were collected using a microplate washer (HYDROFLEX manufactured by Tecan Group, Ltd.), the particles after reaction were washed five times with a washing buffer (TBS including 0.1% by mass of Tween 20). In addition, 50 µL of TBS was added thereto, the mixture was stirred at 25°C for 5 minutes, and next, while the magnetic particles were collected using a microplate washer (HYDROFLEX manufactured by Tecan Group, Ltd.), the particles after reaction were washed five times with a washing buffer (TBS including 0.1% by mass of Tween 20). Next, 50 µL of a luminescent substrate (manufactured by LSI Medience Corporation, substrate solution (R5), CDP-Star) was added thereto, and after 5 minutes, the emission intensity was measured using a luminescence measuring machine (GloMax (registered trademark) Discover System manufactured by Promega Corporation).

### [Measurement method B]

### (Measurement of emission intensity by CLEIA method in EpCAM/CSV evaluation system)

Each heparin plasma specimen was appropriately diluted in advance with a 50 mM Tris buffer containing 2% (w/v) BSA and 150 mM NaCl, and this was used as a sample solution. Onto a 96-well white microplate (manufactured by Corning, Inc.), 25 µL/well of a dispersion liquid of 0.05% (w/v) antibody-bound magnetic particles (Magnosphere MS300/Carboxyl (product No.: MSP-S300-CA, manufactured by JSR Life Sciences, LLC) was sensitized with the antibody by JSR Corporation) having an anti-EpCAM antibody (manufactured by JSR Corporation) bound thereto was added, and next, 25 µL/well of a sample solution was added thereto. In addition, 25 µL/well of a solution containing an alkaline phosphatase-labeled anti-CSV antibody (catalogue No.: H00007431-M08, product manufactured by Abnova Corporation was labeled by JSR Corporation) was added thereto. Subsequently, the 96-well white microplate was stirred at 25°C for 20 minutes, and next, while magnetic particles were collected using a microplate washer (HYDROFLEX manufactured by Tecan Group, Ltd.), the particles after reaction were washed five times with a washing buffer (TBS including 0.1% by mass of Tween 20). In addition, 50 µL of TBS was added thereto, the mixture was stirred at 25°C for 5 minutes, and next, while the magnetic particles were collected using a microplate washer (HYDROFLEX manufactured by Tecan Group, Ltd.), the particles after reaction were washed five times with a washing buffer (TBS including 0.1% by mass of Tween 20). Next, 50 µL/well of a luminescent substrate (manufactured by LSI Medience Corporation, substrate solution (R5), CDP-Star) was added thereto, and after 5 minutes, the emission intensity was measured using a luminescence measuring machine (GloMax (registered trademark) Discover System manufactured by Promega Corporation).

### [Measurement method C]

### (Measurement of emission intensity by CLEIA method in CSV/PD-L1 evaluation system)

Each heparin plasma specimen was appropriately diluted in advance with a 50 mM Tris buffer containing 2% (w/v) BSA and 150 mM NaCl, and this was used as a sample solution. Onto a 96-well white microplate (manufactured by Corning, Inc.), 25 µL of a dispersion liquid of 0.05% (w/v) antibody-bound magnetic particles (Magnosphere MS300/Carboxyl (product No.: MSP-S300-CA, manufactured by JSR Life Sciences, LLC) was sensitized with the antibody by JSR Corporation) having an anti-CSV antibody (catalogue No.: H00007431-M08, manufactured by Abnova Corporation) bound thereto was added, and next, 25 µL of a sample solution was added thereto. In addition, 25 µL/well of a solution containing an alkaline phosphatase-labeled anti-PD-Ll antibody (antibody manufactured by MBL International Corporation was labeled by JSR Corporation) was added thereto. Subsequently, the 96-well white microplate was stirred at 25°C for 20 minutes, and next, while magnetic particles were collected using a microplate washer (HYDROFLEX manufactured by Tecan Group, Ltd.), the particles after reaction were washed five times with a washing buffer (TBS including 0.1% by mass of Tween 20). In addition, 50 µL of TBS was added thereto, the mixture was stirred at 25°C for 5 minutes, and next, while the magnetic particles were collected using a microplate washer (HYDROFLEX manufactured by Tecan Group, Ltd.), the particles after reaction were washed five times with a washing buffer (TBS including 0.1% by mass of Tween 20). Next, 50 µL/well of a luminescent substrate (manufactured by LSI Medience Corporation, substrate solution (R5), CDP-Star) was added thereto, and after 5 minutes, the emission intensity was measured using a luminescence measuring machine (GloMax (registered trademark) Discover System manufactured by Promega Corporation).

### <Measurement of CSV-positive exosomes in lung cancer patient specimen>

### [Example 1]

Heparin plasma specimens (47 cases) of lung cancer patients including early-stage lung cancer patients of stage 1 or 2 were used as sample solutions, and for each of the specimens, measurement was performed according to the above-described measurement method A. A value (S/N: Signal/Noise ratio) obtained by dividing the measured value of each sample by the blank value was calculated.

### [Comparative Example 1]

Heparin plasma specimens (8 cases) of healthy subjects were used as sample solutions, and for each of them, measurement was performed according to the above-described measurement method A. A value (S/N) obtained by dividing the measured value of each sample by the blank value was calculated.

### [Analysis Example 1]

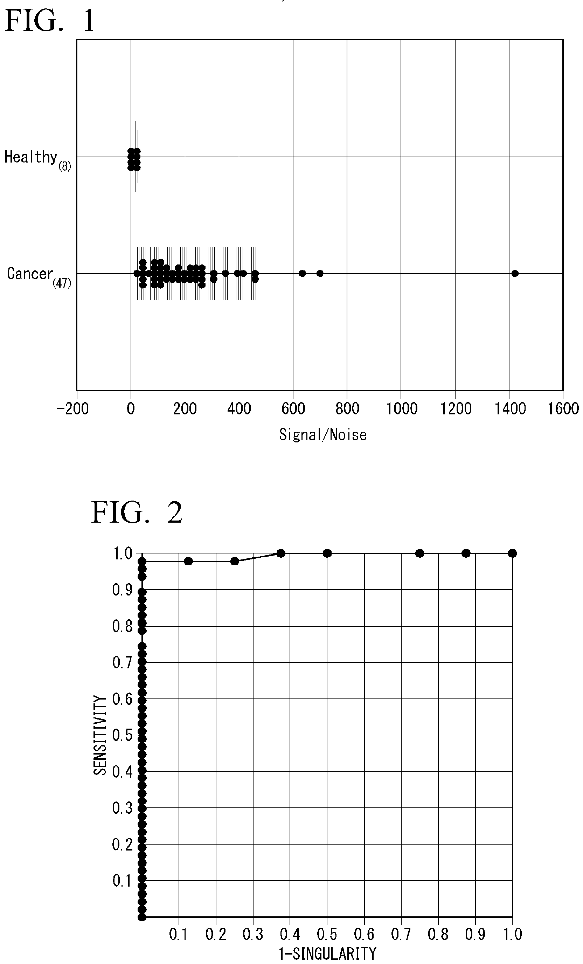

The results of Example 1 and Comparative Example 1 are shown in Fig. 1. In Fig. 1, "Healthy" denotes heparin plasma specimens of healthy subjects, and "Cancer" denotes heparin plasma specimens of lung cancer patients. The same applies to the subsequent drawings. Furthermore, an ROC curve created from the measurement results shown in Fig. 1 is shown in Fig. 2.

Verification was performed by taking the S/N of healthy subjects obtained in Comparative Example 1 as a control group and taking the S/N of lung cancer patients obtained in Example 1 as a group to be determined, and when the cutoff value was set to 29.47, the sensitivity (specificity) was 0.98, suggesting that lung cancer patients can be distinguished from healthy subjects.

### [Example 2]

Heparin plasma specimens (47 cases) of lung cancer patients including early-stage lung cancer patients of stage 1 or 2 were used as sample solutions, and for each of the specimens, measurement was performed according to the above-described measurement method B. A value (S/N) obtained by dividing the measured value of each sample by the blank value was calculated.

### [Comparative Example 2]

Heparin plasma specimens (8 cases) of healthy subjects were used as sample solutions, and for each of them, measurement was performed according to the above-described measurement method B. A value (S/N) obtained by dividing the measured value of each sample by the blank value was calculated.

### [Analysis Example 2]

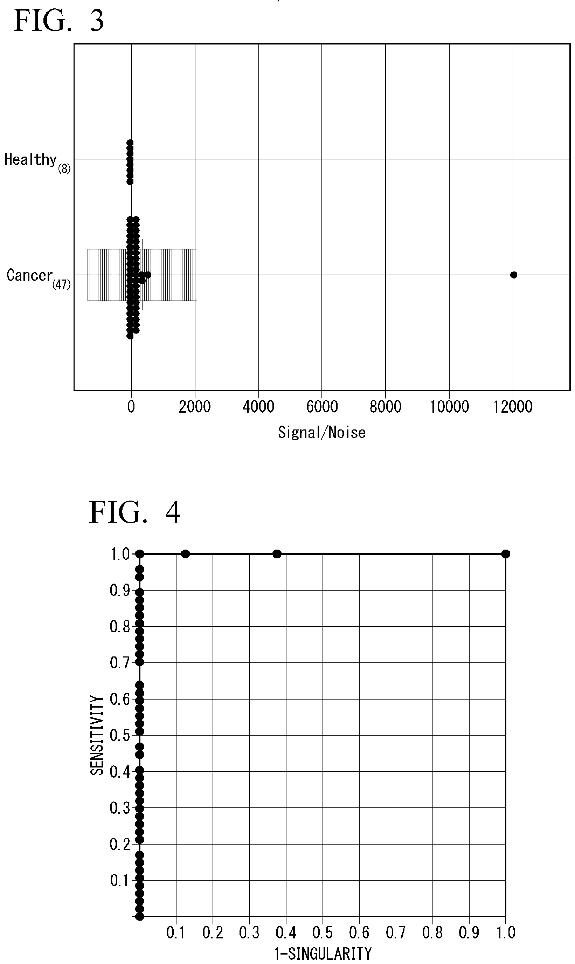

The results of Example 2 and Comparative Example 2 are shown in Fig. 3. Furthermore, an ROC curve created from the measurement results shown in Fig. 3 is shown in Fig. 4.

Verification was performed by taking the S/N of healthy subjects obtained in Comparative Example 2 as a control group and taking the S/N of lung cancer patients obtained in Example 2 as a group to be determined, and when the cutoff value was set to 4.00, the sensitivity (specificity) was 1.00, suggesting that lung cancer patients can be distinguished from healthy subjects.

### [Example 3]

Heparin plasma specimens (47 cases) of lung cancer patients including early-stage lung cancer patients of stage 1 or 2 were used as sample solutions, and for each of the specimens, measurement was performed according to the above-described measurement method C. A value (S/N) obtained by dividing the measured value of each sample by the blank value was calculated.

### [Comparative Example 3]

Heparin plasma specimens (8 cases) of healthy subjects were used as sample solutions, and for each of them, measurement was performed according to the above-described measurement method C. A value (S/N) obtained by dividing the measured value of each sample by the blank value was calculated.

### [Analysis Example 3]

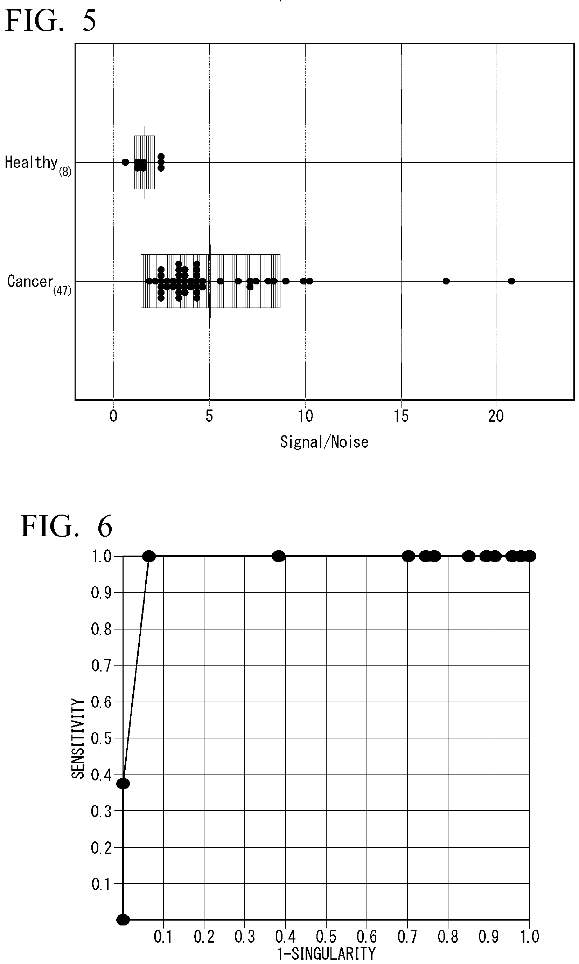

The results of Example 3 and Comparative Example 3 are shown in Fig. 5. Furthermore, an ROC curve created from the measurement results shown in Fig. 5 is shown in Fig. 6.

Verification was performed by taking the S/N of healthy subjects obtained in Comparative Example 3 as a control group and taking the S/N of lung cancer patients obtained in Example 3 as a group to be determined, and when the cutoff value was set to 2.91, the sensitivity (specificity) was 0.94, suggesting that lung cancer patients can be distinguished from healthy subjects.

### [Analysis Example 4]

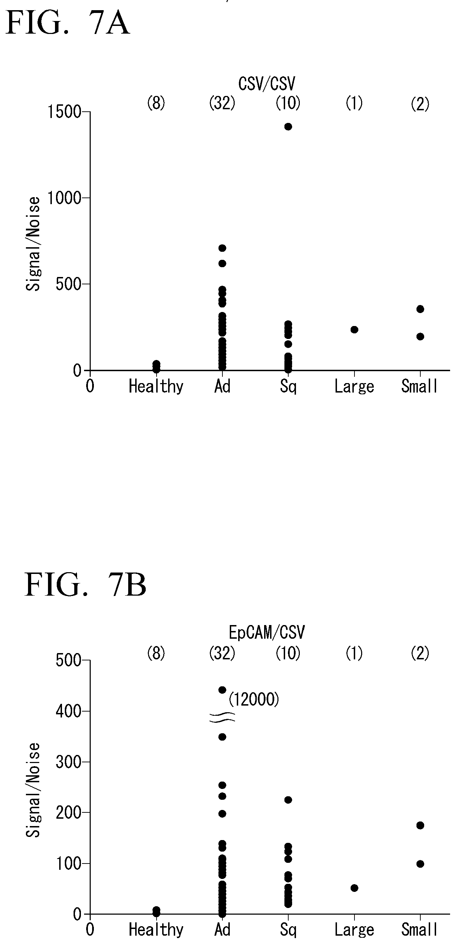

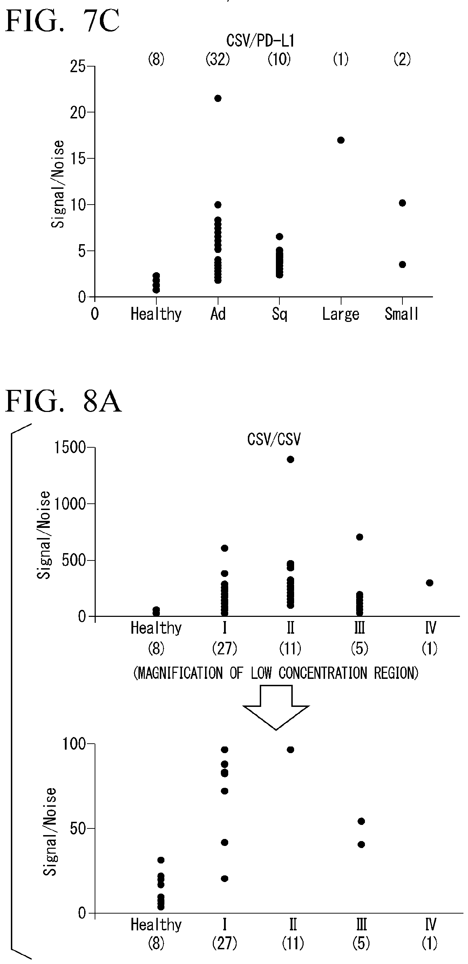

The measurement results of Example 1 and Comparative Example 1, Example 2 and Comparative Example 2, Example 3 and Comparative Example 3 were each classified according to the type of lung cancer. The results are shown in Fig. 7A (Example 1 and Comparative Example 1: CSV/CSV evaluation system), Fig. 7B (Example 2 and Comparative Example 2: EpCAM/CSV evaluation system), and Fig. 7C (Example 3 and Comparative Example 3: CSV/PD-L1 evaluation system). In Fig. 7A to Fig. 7C, "Ad" is an abbreviation for Adenocarcinoma, which is adenocarcinoma. "Sq" is an abbreviation for Squamous, which is squamous cell carcinoma. "Large" denotes large cell carcinoma. "Small" denotes a small cell lung cancer.

In Fig. 7A to Fig. 7B, the S/N of lung cancer patients exhibited higher values than the S/N of healthy subjects in all cancer types.

### [Analysis Example 5]

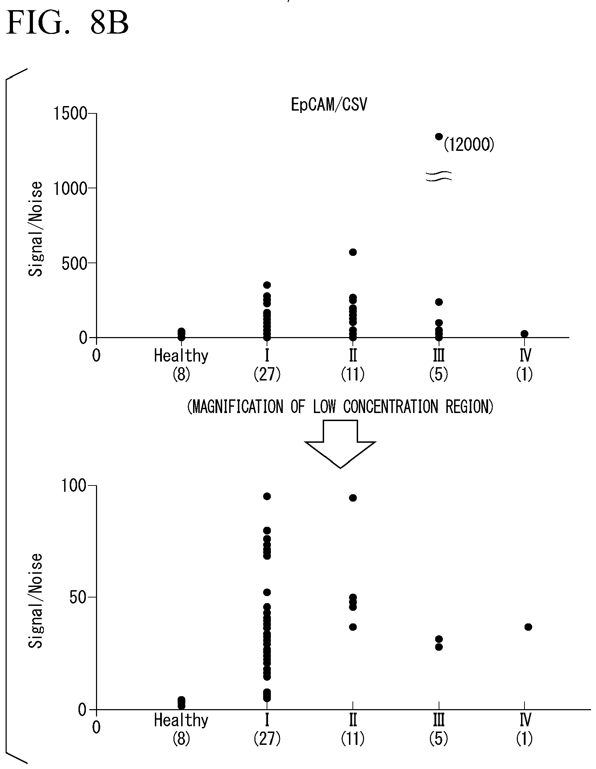

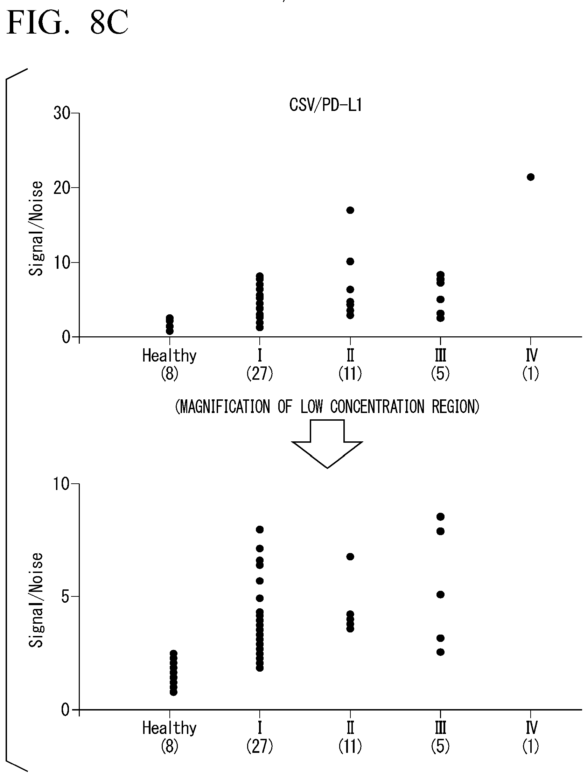

The measurement results of Example 1 and Comparative Example 1, Example 2 and Comparative Example 2, Example 3 and Comparative Example 3 were classified according to the pathological stage. The results are shown in Fig. 8A (Example 1 and Comparative Example 1: CSV/CSV evaluation system), Fig. 8B (Example 2 and Comparative Example 2: EpCAM/CSV evaluation system), and Fig. 8C (Example 3 and Comparative Example 3: CSV/PD-L1 evaluation system). In Fig. 8A to Fig. 8C, the pathological stages are as follows. The same applies to the subsequent drawings.

### (Pathological stage)

Japan Lung Cancer Society, ed.: Lung Cancer Handling Regulations, 8th Edition, TNM clinical staging was followed.

From Fig. 8A to Fig. 8C, the S/N of lung cancer patients had higher values than the S/N of healthy subjects from the early stage of the pathological stage, and lung cancer patients could be distinguished from healthy subjects irrespective of the pathological stage from stage I to stage IV.

### [Analysis Example 6]

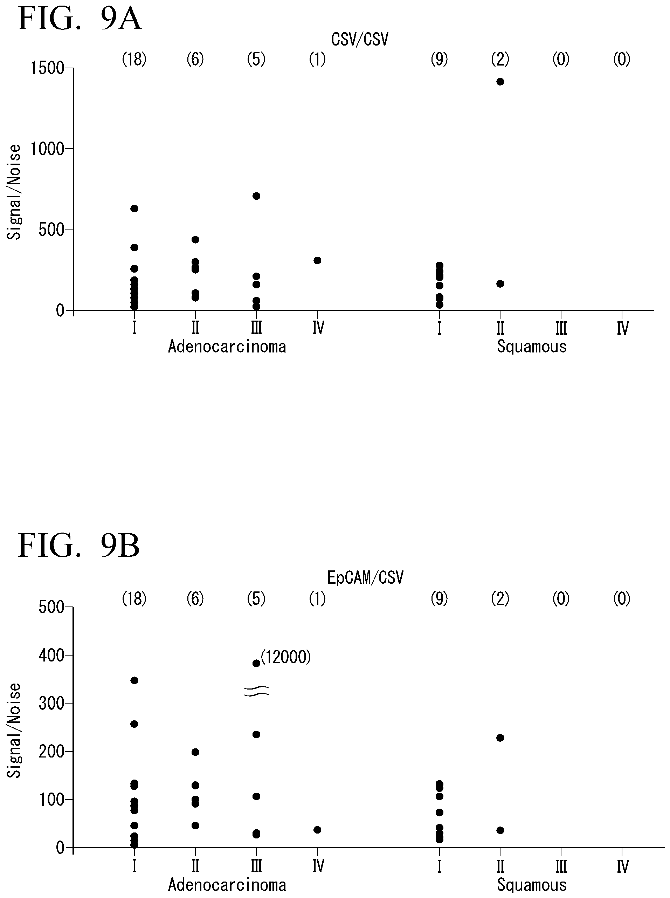

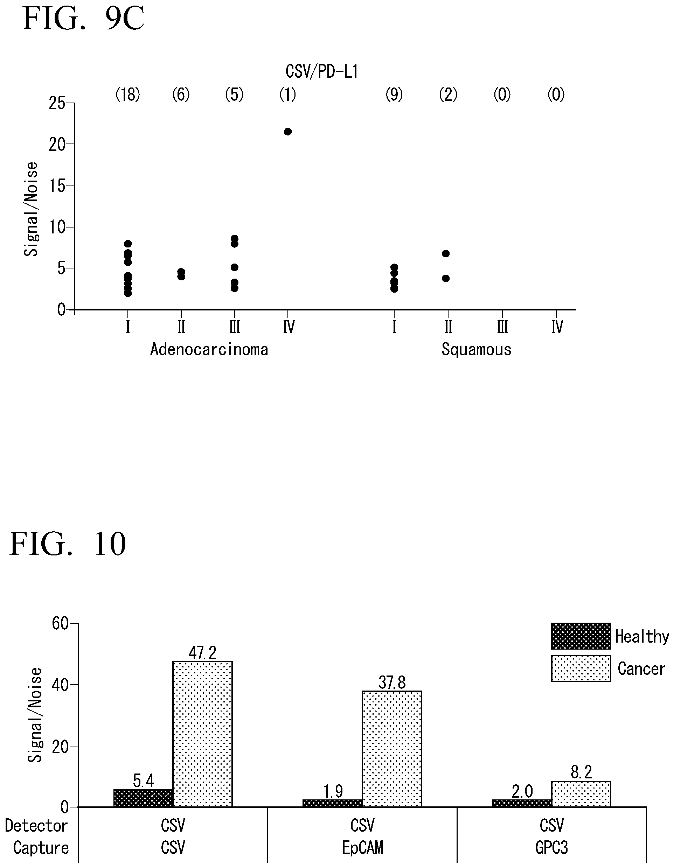

The measurement results of adenocarcinoma patients and squamous cell carcinoma patients among the measurement results of Example 1 and Comparative Example 1, Example 2 and Comparative Example 2, Example 3 and Comparative Example 3 were classified according to the pathological stage. The results are shown in Fig. 9A (Example 1 and Comparative Example 1: CSV/CSV evaluation system), Fig. 9B (Example 2 and Comparative Example 2: EpCAM/CSV evaluation system), and Fig. 9C (Example 3 and Comparative Example 3: CSV/PD-L1 evaluation system).

From Fig. 9A to Fig. 9C, regarding the S/N of adenocarcinoma patients and squamous cell carcinoma patients, the values tended to increase from the early stage of the pathological stage. This suggests that adenocarcinoma patients and squamous cell carcinoma patients can be distinguished from healthy subjects by an evaluation system that combines an antibody against an antigen expressed on a cancer cell surface and an anti-CSV antibody.

Furthermore, regarding the heparin plasma specimens of lung cancer patients, the concentrations of carcinoembryonic antigen (CEA), squamous cell carcinoma-related antigen (SCC), sialyl Le^{x}-i antigen (SLX), nerve-specific enolase (NSE), gastrin releasing peptide precursor (Pro GRP), and cytokeratin 19 fragment (Cyfra), which are existing lung cancer markers, were measured. Next, for the CSV/CSV evaluation systems of Example 1 and Comparative Example 1, the EpCAM/CSV evaluation systems of Example 2 and Comparative Example 2, and the CSV/PD-L1 evaluation systems of Example 3 and Comparative Example 3, the correlation of each of the evaluation systems with the above-described existing lung cancer markers was examined; however, no correlation was found.

In addition, the CSV/CSV evaluation systems of Example 1 and Comparative Example 1, the EpCAM/CSV evaluation systems of Example 2 and Comparative Example 2, and the CSV/PD-L1 evaluation systems of Example 3 and Comparative Example 3, the correlation between each of the evaluation systems was examined; however, no correlation was found.

In the examination of the above-described correlation, examination was performed after the specimen for which the S/N of the EpCAM/CSV evaluation system was 12000 was excluded as an abnormal value.

From these facts, the accuracy of cancer diagnosis can be enhanced by performing diagnosis by combining existing markers and the present evaluation system or a plurality of the present evaluation systems.

### [Example 4]

Each of the heparin plasma specimens of healthy subjects and the heparin pool plasma specimens of lung cancer patients was appropriately diluted in advance with 50 mM Tris buffer containing 2% (w/v) BSA and 150 mM NaCl, and a sample solution was obtained. Onto a 96-well white microplate (manufactured by Corning, Inc.), 25 µL/well each of dispersion liquids (three kinds) of 0.05% (w/v) antibody-bound magnetic particles (Magnosphere MS300/Carboxyl (product No.: MSP-S300-CA, manufactured by JSR Life Sciences, LLC) was sensitized with the antibody by JSR Corporation) having any one of an anti-CSV antibody (catalogue No.: H00007431-M08, manufactured by Abnova Corporation), an anti-EpCAM antibody (manufactured by JSR Corporation), or an anti-glypican-3 antibody (anti-GPC3 antibody) (manufactured by MBL International Corporation) bound thereto was added, and next, 25 µL/well of a sample solution was added thereto. In addition, 25 µL/well of a solution containing an alkaline phosphatase-labeled anti-CSV antibody (catalogue No.: H00007431-M08, product manufactured by Abnova Corporation was labeled by JSR Corporation) was added thereto. Subsequently, the 96-well white microplate was stirred at 25°C for 20 minutes, and next, while magnetic particles were collected using a microplate washer (HYDROFLEX manufactured by Tecan Group, Ltd.), the particles after reaction were washed five times with a washing buffer (TBS including 0.1% by mass of Tween 20). In addition, 50 µL of TBS was added thereto, the mixture was stirred at 25°C for 5 minutes, and next, while the magnetic particles were collected using a microplate washer (HYDROFLEX manufactured by Tecan Group, Ltd.), the particles after reaction were washed five times with a washing buffer (TBS including 0.1% by mass of Tween 20). Next, 50 µL/well of a luminescent substrate (manufactured by LSI Medience Corporation, substrate solution (R5), CDP-Star) was added thereto, and after 5 minutes, the emission intensity was measured using a luminescence measuring machine (GloMax (registered trademark) Discover System manufactured by Promega Corporation). A value (S/N) obtained by dividing the measured value of each sample by the blank value was calculated. The results are shown in Fig. 10. In Fig. 10, "Capture" denotes a capture molecule, and "Detector" denotes a detector molecule.

From Fig. 10, it was suggested that since an evaluation system could be constructed even by using the co-expression of GPC3 and CSV, which are cancer antigens different from EpCAM, as a marker, lung cancer patients can be distinguished from healthy subjects by means of an evaluation system combining an antibody against an antigen expressed on a cancer cell surface and an anti-CSV antibody.

### [Example 5]

A heparin plasma specimen of a healthy subject was diluted in advance with a 50 mM Tris buffer containing 2% (w/v) BSA and 150 mM NaCl, and this was used as a sample solution. Onto a 96-well white microplate (manufactured by Corning, Inc.), 25 µL/well of a dispersion liquid of 0.05% (w/v) antibody-bound magnetic particles (Magnosphere MS300/Carboxyl (product No.: MSP-S300-CA, manufactured by JSR Life Sciences, LLC) was sensitized with the antibody by JSR Corporation) having an anti-PD-Ll antibody (manufactured by MBL International Corporation) bound thereto was added, and next, 25 µL/well of a sample solution was added thereto. In addition, 25 µL/well of a solution of an alkaline phosphatase-labeled anti-CSV antibody (catalogue No.: H00007431-M08, product manufactured by Abnova Corporation was labeled by JSR Corporation) was added thereto. Subsequently, the 96-well white microplate was stirred at 25°C for 20 minutes, and next, while magnetic particles were collected using a microplate washer (HYDROFLEX manufactured by Tecan Group, Ltd.), the particles after reaction were washed five times with a washing buffer (TBS including 0.1% by mass of Tween 20). In addition, 50 µL of TBS was added thereto, the mixture was stirred at 25°C for 5 minutes, and next, while the magnetic particles were collected using a microplate washer (HYDROFLEX manufactured by Tecan Group, Ltd.), the particles after reaction were washed five times with a washing buffer (TBS including 0.1% by mass of Tween 20). Next, 50 µL/well of a luminescent substrate (manufactured by LSI Medience Corporation, substrate solution (R5), CDP-Star) was added thereto, and after 5 minutes, the emission intensity was measured using a luminescence measuring machine (GloMax (registered trademark) Discover System manufactured by Promega Corporation) (hereinafter, this evaluation system may be referred to as "CSV/PD-L1 evaluation system").

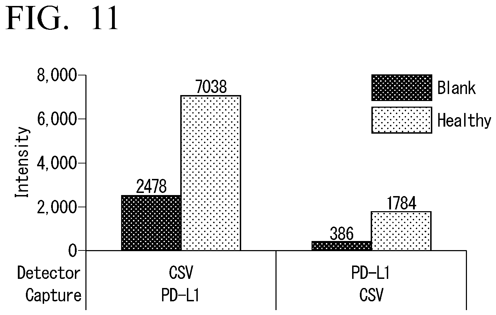

Apart from this, the emission intensity was measured using a method similar to the method used for the "CSV/PD-L1 evaluation system", except that a dispersion liquid of 0.05% (w/v) antibody-bound magnetic particles (Magnosphere MS300/Carboxyl (product No.: MSP-S300-CA, manufactured by JSR Life Sciences, LLC) was sensitized with the antibody by JSR Corporation) having an anti-CSV antibody (catalogue No.: H00007431-M08, manufactured by Abnova Corporation) bound thereto was used instead of a dispersion liquid of 0.05% (w/v) antibody-bound magnetic particles (Magnosphere MS300/Carboxyl (product No.: MSP-S300-CA, manufactured by JSR Life Sciences, LLC) was sensitized with the antibody by JSR Corporation) having an anti-PD-Ll antibody (manufactured by MBL International Corporation) bound thereto, and an alkaline phosphatase-labeled anti-PD-Ll antibody (an antibody manufactured by MBL International Corporation was labeled by JSR Corporation) was used instead of the alkaline phosphatase-labeled anti-CSV antibody (catalogue No.: H00007431-M08, product manufactured by Abnova Corporation was labeled by JSR Corporation) (hereinafter, this evaluation system may be referred to as "PD-L1/CSV evaluation system"). The results are shown in Fig. 11. In Fig. 11, "Capture" denotes a capture molecule, and "Detector" denotes a detector molecule.

From Fig. 11, the intensities of the "CSV/PD-L1 evaluation system" and the "PD-L1/CSV evaluation system" were compared, and it was suggested that an evaluation system can be constructed even when the combination of the capture molecule and the detector molecule is reversed.

### INDUSTRIAL APPLICABILITY

According to the method for separation and detection of exosomes and the kit for separation and detection of the present embodiment, exosomes can be separated and can be utilized for cancer diagnosis and companion diagnostics.

## Claims

1. A method for separation and detection of exosomes, the method comprising:
a complex formation step of bringing a biological sample into contact with a capture molecule, the capture molecule including a specific binding substance for an antigen expressed on a cancer cell surface, to form a complex of an exosome and the capture molecule; and
a detection step of bringing the complex into contact with a detector molecule, the detector molecule including a specific binding substance for an antigen expressed on a cancer cell surface and a labeling substance, to detect the complex by using the detector molecule,
wherein the antigen expressed on a cancer cell surface for at least one of the capture molecule and the detector molecule is cell-surface vimentin.

2. The method according to Claim 1, wherein the specific binding substance for the cell-surface vimentin is an anti-CSV antibody.

3. The method according to Claim 1 or 2, wherein the specific binding substance for the antigen expressed on a cancer cell surface in the capture molecule is an anti-CSV antibody, and the specific binding substance for the antigen expressed on a cancer cell surface in the detector molecule is an anti-CSV antibody, an anti-EpCAM antibody, or an anti-PD-Ll antibody.

4. The method according to Claim 1 or 2, wherein the specific binding substance for the antigen expressed on a cancer cell surface in the detector molecule is an anti-CSV antibody, and the specific binding substance for the antigen expressed on a cancer cell surface in the capture molecule is an anti-CSV antibody, an anti-EpCAM antibody, or an anti-PD-Ll antibody.

5. The method according to any one of Claims 1 to 4, wherein the capture molecule is immobilized on a magnetic particle.

6. The method according to any one of Claims 1 to 5, the method further comprising, after the complex formation step and before the detection step,
a washing step of adding a washing liquid to remove foreign substances other than the complex.

7. The method according to any one of Claims 1 to 6, wherein in the detection step, the complex is brought into contact with the detector molecule, subsequently a washing liquid is added to remove foreign substances other than the complex to which the detector molecule is bound, and then the complex is detected by using the detector molecule.

8. A kit for separation and detection of exosomes, the kit comprising:
a capture molecule including a specific binding substance for an antigen expressed on a cancer cell surface; and
a detector molecule including a specific binding substance for an antigen expressed on a cancer cell surface and a labeling substance,
wherein the antigen expressed on a cancer cell surface for at least one of the capture molecule and the detector molecule is cell-surface vimentin.

9. The kit according to Claim 8, wherein the specific binding substance for the antigen expressed on cancer cell surface is an anti-CSV antibody.

10. The kit according to Claim 8 or 9, wherein the specific binding substance for the antigen expressed on a cancer cell surface in the capture molecule is an anti-CSV antibody, and the specific binding substance for the antigen expressed on a cancer cell surface in the detector molecule is an anti-CSV antibody, an anti-EpCAM antibody, or an anti-PD-Ll antibody.

11. The kit according to Claim 8 or 9, wherein the specific binding substance for the antigen expressed on a cancer cell surface in the detector molecule is an anti-CSV antibody, and the specific binding substance for the antigen expressed on a cancer cell surface in the capture molecule is an anti-CSV antibody, an anti-EpCAM antibody, or an anti-PD-Ll antibody.

12. The kit according to any one of Claims 8 to 11, wherein the capture molecule is immobilized on a magnetic particle.

13. The kit according to any one of Claims 8 to 12, wherein the kit is used for diagnosing cancer.

14. The kit according to any one of Claims 8 to 13, wherein the kit is used for diagnosing early-stage cancer.

15. The kit according to any one of Claims 8 to 14, wherein the kit is used for diagnosing lung cancer.

16. The kit according to any one of Claims 8 to 12, wherein the kit is used for companion diagnostics.
